# EUROPÄISCHE PATENTSCHRIFT

(11) **EP 0 251 079 B1**
(45) Veröffentlichungstag und Bekanntmachung des Hinweises auf die Patenterteilung: **19.05.1993**
(21) Anmeldenummer: 87108837.3
(22) Anmeldetag: 19.06.1987
(51) Int. Cl.: C07D 251/52, C07D 239/42, C07D 251/46, C07D 239/52, C07D 251/42, C07D 253/06, A01N 47/34

(54) **3-Substituierte 1-(2-Halogen-alkoxy-benzolsulfonyl)-3-heteroaryl-harnstoffe**
3-Substituted 1-(2-halogenoalkoxy-benzensulfonyl)-3-heteroarylurea
Urées, 3-substituées, 1-(2-halogénoalcoxybenzène-sulfonyl-3-hétéroaryle

(30) Priorität: 26.06.1986 DE 3621320
(43) Veröffentlichungstag der Anmeldung: 07.01.1988
(73) Patentinhaber: BAYER AG, 51368 Leverkusen (DE)
(72) Erfinder: Pfister, Theodor, Dr., D-4019 Monheim (DE); Fest, Christa, Dr., D-5600 Wuppertal 1 (DE); Müller, Klaus-Helmut, Dr., D-4000 Düsseldorf 13 (DE); Riebel, Hans-Jochem, Dr., D-5600 Wuppertal 1 (DE); Santel, Hans-Joachim, Dr., D-5090 Leverkusen 1 (DE); Schmidt, Robert R, Dr., D-5060 Bergisch-Gladbach 2 (DE)

(56) Entgegenhaltungen:
- EP-A- 0 048 143
- EP-A- 0 108 708
- EP-A- 0 128 274
- EP-A- 0 152 378
- EP-A- 0 173 312
- DE-A- 3 234 448
- CHEMICAL ABSTRACTS, Band 106, 1987, Seite 695, Zusammenfassung Nr. 156500f, Columbus, Ohio, US; M. CONNER: "Herbicidal sulfonamides"

## Beschreibung

Die Erfindung betrifft neue 3-Methyl-3-(1,3,5-triazin-2-yl)-1-(2-fluormethoxy-benzolsulfonyl)-harnstoffe, mehrere Verfahren zu ihrer Herstellung und ihre Verwendung als Herbizide.

Es ist bereits bekannt, daß bestimmte 3-Heteroaryl-1-arylsulfonyl-harnstoffe (kurz: "Sulfonylharnstoffe") herbizid wirksam sind. Aus dem umfangreichen Stand der Technik hierzu sollen folgende Verbindungsgruppen beispielhaft genannt werden:
Sulfonylharnstoffe, die in 3-Stellung außer dem Heteroarylrest nur Wasserstoff tragen (vgl. EP-A-0 173 312 sowie US-A-4 169 719 und DE-A-3 234 448);
Phenylsulfonylharnstoffe mit einem Alkylsulfonylsubstituenten in 2-Stellung der Phenylgruppe (vgl. EP-A-0 048 143);
Sulfonylharnstoffe mit einem Cyclopropylsubstituenten im Heteroarylring (vgl. EP-A-0 108 708);
ferner Sulfonylharnstoffe, die in 3-Stellung außer dem Heteroarylrest - gegebenenfalls substituierte - Allyl- oder Propargylgruppen (vgl. EP-A-0 128 274) oder eine durch schwefel- oder stickstoffhaltige Reste substituierte Methylgruppe tragen (vgl. EP-A-0 152 378).

Als Verbindungen aus dem genannten Stand der Technik, welche den erfindungsgemäßen Wirkstoffen strukturell besonders nahekommen, sind z.B. 3-(4,6-Dimethoxy-1,3,5-triazin-2-yl)-1-(2-trifluormethoxy-benzolsulfonyl)-harnstoff und 3-(4-Methoxy-6-methyl-1,3,5-triazin-2-yl)-1-(2-trifluormethoxy-benzolsulfonyl)-harnstoff zu nennen (vgl. EP-A-0 173 312); die Wirkung dieser Verbindungen ist jedoch nicht immer ganz befriedigend.

In genereller Form umfaßt die Offenbarung gemäß EP-A-0 173 312 auch solche Sulfonylharnstoffe des oben bezeichneten Typs, die in 3-Stellung außer dem Heteroarylrest - an Stelle von Wasserstoff - einen C₁₋₄-Alkyl- oder Benzylrest tragen können. Jedoch wird kein Beispiel einer Verbindung dieser Untergruppe von 3-alkylierten Derivaten konkret beschrieben, so daß z.B. 3-methylsubstituierte 3-(1,3,5-Triazin-2-yl)-1-(2-fluormethoxybenzolsulfonyl)-harnstoffe als solche und ihre tatsächlichen Eigenschaften aus dem Stand der Technik nicht bekannt sind.

Es wurden nun neue 3-Methyl-3-(1,3,5-triazin-2-yl)-1-(2-fluormethoxy-benzolsulfonyl)-harnstoffe der Formel (I), 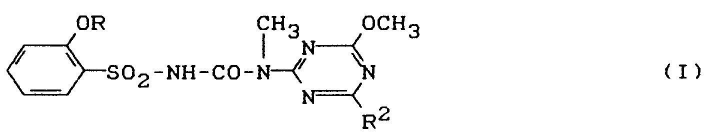
in welcher
- R: für CHF₂ oder CF₃ steht und
- R²: für CH₃ oder OCH₃ steht,
sowie Salze von Verbindungen der Formel (I) mit Natrium, Kalium, Magnesium oder Calcium gefunden.

Man erhält die neuen 3-Methyl-3-(1,3,5-triazin-2-yl)-1-(2-fluormethoxy-benzolsulfonyl)-harnstoffe der Formel (I) und ihre Na-, K-, Mg- und Ca-Salze, wenn man
(a) 2-Fluormethoxy-benzolsulfonyl-isocyanate der Formel (II) 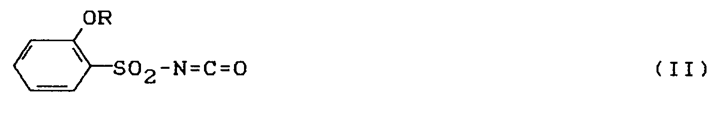 in welcher
   - R: für CHF₂ oder CF₃ steht,
   mit N-Methyl-(1,3,5-triazin-2-yl)-aminen der Formel (III) 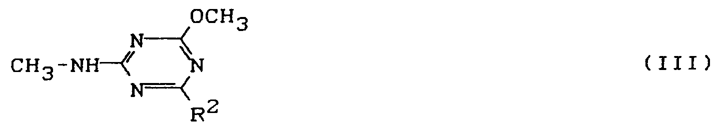 in welcher
   - R²: für CH₃ oder OCH₃ steht,
   gegebenenfalls in Gegenwart von Verdünnungsmitteln und gegebenenfalls in Gegenwart von Katalysatoren umsetzt und gegebenenfalls die so erhaltenen Produkte nach üblichen Methoden in die Na-, K-, Mg- oder Ca-Salze überführt,
   oder wenn man
(b) 2-Fluormethoxy-benzolsulfonsäureamide der Formel (IV), 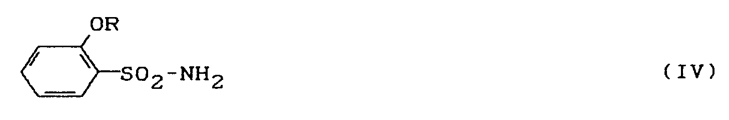 in welcher
   - R: für CHF₂ oder CF₃ steht,
   mit N-Methyl-N-(1,3,5-triazin-2-yl)-urethanen der Formel (V) 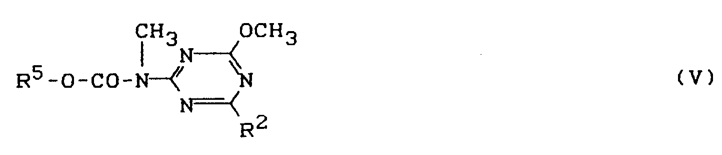 in welcher
   - R²: für CH₃ oder OCH₃ steht und
   - R⁵: für C₁-C₄-Alkyl, Benzyl oder Phenyl steht,
   gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt,
   oder wenn man
(c) N-(2-Fluormethoxy-benzolsulfonyl)-urethane der Formel (VI), 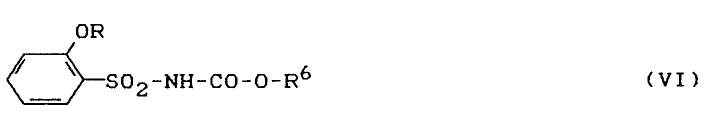 in welcher
   - R: für CHF₂ oder CF₃ steht und
   - R⁶: für C₁-C₄-Alkyl, Benzyl oder Phenyl steht,
   mit N-Methyl-(1,3,5-triazin-2-yl)-aminen der Formel (III), 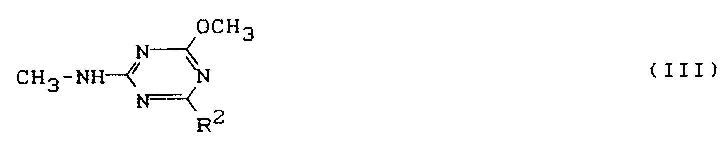 in welcher
   - R²: für CH₃ oder OCH₃ steht,
   gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt,
   oder wenn man
(d) 1-(2-Fluormethoxy-benzolsulfonyl)-3-(1,3,5-triazin-2-yl)-harnstoffe der Formel (VII), 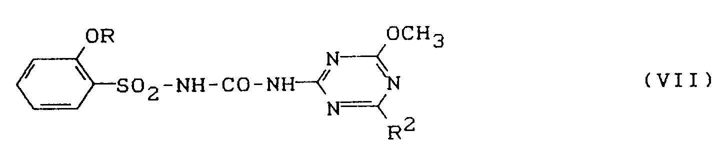 in welcher
   - R: für CHF₂ oder CF₃ steht und
   - R²: für CH₃ oder OCH₃ steht,
   mit Verbindungen (Methylierungsmitteln) der Formel (VIII),

   CH₃-W (VIII)

   in welcher
   - W: für eine nucleophile Abgangsgruppe steht,
   gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt.

Die neuen 3-Methyl-3-(1,3,5-triazin-2-yl)-1-(2-fluormethoxy-benzolsulfonyl)-harnstoffe der Formel (I) zeichnen sich durch starke herbizide Wirksamkeit aus.

Überraschenderweise zeigen die neuen Verbindungen der Formel (I) erheblich bessere herbizide Wirkung als vorbekannte Harnstoff-Derivate gleicher Wirkungsrichtung.

Verwendet man beispielsweise für die Verfahrensvariante
(a) 2-Trifluormethoxy-benzolsulfonyl-isocyanat und 4-Methoxy-6-methyl-2-methylamino-1,3,5-triazin als Ausgangsstoffe, so kann der Reaktionsablauf durch folgendes Formelschema skizziert werden: 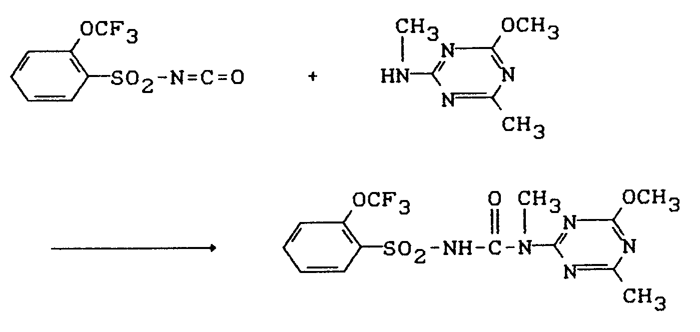 Verwendet man beispielsweise für die Verfahrensvariante
(b) 2-Difluormethoxy-benzolsulfonsäureamid und O-Phenyl-N-methyl-N-(4-methoxy-6-methyl-1,3,5-triazin-2-yl)-urethan als Ausgangsstoffe, so kann der Reaktionsablauf durch das folgende Formelschema skizziert werden: 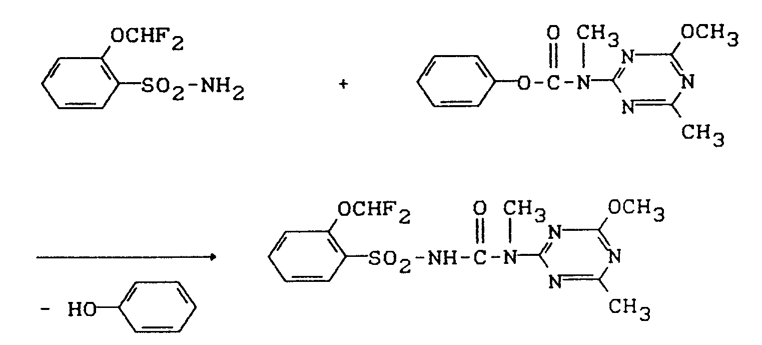 Verwendet man beispielsweise für die Verfahrensvariante
(c) O-Phenyl-N-(2-difluormethoxy-benzolsulfonyl)-urethan und 2-Methylamino-4,6-dimethoxy-1,3,5-triazin als Ausgangsstoffe, so kann der Reaktionsablauf durch das folgende Formelschema skizziert werden: 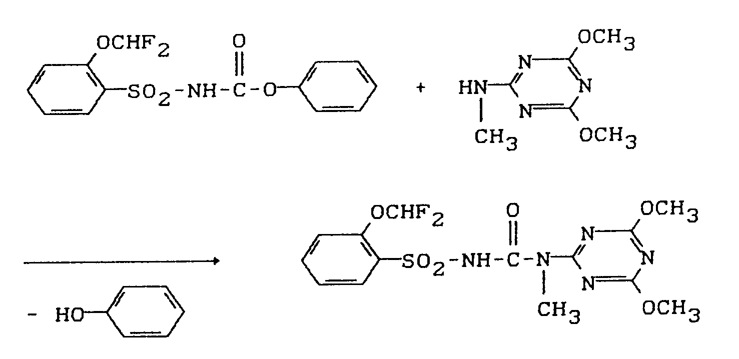 Verwendet man beispielsweise für die Verfahrensvariante
(d) 3-(4,6-Dimethoxy-1,3,5-triazin-2-yl)-1-(2-trifluormethoxy-benzolsulfonyl)-harnstoff und Methyliodid als Ausgangsstoffe, so kann der Reaktionsablauf durch das folgende Formelschema skizziert werden:

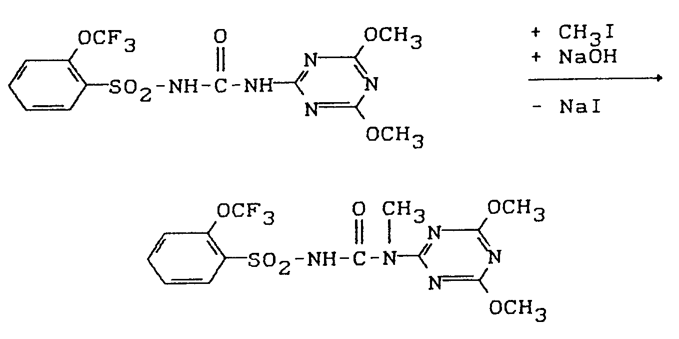

Die bei der Verfahrensvariante (a) als Ausgangsstoffe zu verwendenden 2-Fluormethoxy-benzolsulfonyl-isocyanate der Formel (II) sind bekannt und lassen sich nach bekannten Verfahren herstellen (vergl. z.B. EP-A 44 808 und EP-A 173 312 und US-PS 4 514 212).

Die bei der Verfahrensvariante (a) außerdem als Ausgangsstoffe zu verwendenden N-Methyl-(1,3,5-triazin-2-yl)-amine der Formel (III) sind gleichfalls bekannt und können nach an sich bekannten Verfahren hergestellt werden (vergl. z.B. Chem. Pharm. Bull. 11 (1963), 1382 und US-PS 4 299 960, EP-A 121 082, 125 205, 126 711 und EP-A 152 378).

Die bei der Verfahrensvariante (b) als Ausgangsstoffe zu verwendenden 2-Fluormethoxy-benzolsulfonsäureamide der Formel (IV) sind bekannt (vergl. z.B. Zh. Org. Khim, [J. Org. Chem. USSR], 8, (1972) 1023 - 1026 [engl. 1032 - 1034] und EP-A 44 808).

Die bei der Verfahrensvariante (b) außerdem als Ausgangsstoffe zu verwendenden N-Methyl-N-(1,3,5-triazin-2-yl)-urethane der Formel (V) sind ebenfalls bekannt und können nach an sich bekannten Verfahren hergestellt werden (vergl. z.B. EP-A 101 670, 121 082, 125 205, 126 711 und EP-A 152 378).

Die bei der Verfahrensvariante (c) als Ausgangsstoffe zu verwendenden N-(2-Fluormethoxy-benzolsulfonyl)-urethane der Formel (VI) sind bekannt (vergl. z.B. EP-A 125 205, 121 082, 44 808 und EP-A 44 809).

Auch die bei der Verfahrensvariante (d) als Ausgangsstoffe zu verwendenden 1-(2-Fluormethoxy-benzolsulfonyl)-3-(1,3,5-triazin-2-yl)-harnstoffe der Formel (VII) sind bekannt und lassen sich nach bekannten Methoden herstellen (vergl. z.B. EP-A 44 808, 44 809 und EP-A 173 312).

Die bei der Verfahrensvariante (d) außerdem als Ausgangsstoffe zu verwendenden - allgemein bekannten - Methylierungsmittel sind durch die Formel (VIII) allgemein definiert. Die nucleophile Abgangsgruppe W steht in Formel (VIII) vorzugsweise für Chlorid, Bromid, Iodid,
-O-SO₂-OCH₃, -O-SO₂CH₃ und

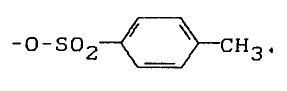

Bevorzugte Methylierungsmittel gemäß Formel (VIII) sind somit Methylchlorid, Methylbromid, Methyliodid, sowie Dimetylsulfat, Methansulfonsäuremethylester und p-Toluolsulfonsäuremethylester.

Das erfindungsgemäße Verfahen (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) wird gegebenenfalls in Gegenwart eines Katalysators durchgeführt. Hierzu gehören insbesondere aliphatische, aromatische oder heterocyclische Amine, wie Triethylamin, N,N-Dimethylanilin, Pyridin, 2-Methyl-5-ethyl-pyridin, 4-Dimethylamino-pyridin, Diazabicyclooctan (DABCO), Diazabicyclononen (DBN) und Diazabicycloundecen (DBU).

Die Reaktionstemperaturen können bei dem erfindungsgemäßen Verfahren (a) in einem größeren Bereich variiert werden Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man je Mol Ausgangsstoff der Formel (III) im allgemeinen 1,0 bis 3,0 Mol, vorzugsweise 1,0 bis 2,0 Mol Ausgangsstoff der Formel (II) ein.

Die Ausgangsstoffe der Formeln (II) und (III) und gegebenenfalls der Katalysator und das Verdünnungsmittel werden im allgemeinen bei Raumtemperatur oder unter leichter Aussenkühlung zusammengegeben und das Reaktionsgemisch wird gegebenenfalls bei erhöhter Temperatur bis zum Reaktionsende gerührt.

Die Aufarbeitung und Isolierung der neuen Verbindungen der Formel (I) erfolgt nach üblichen Methoden.

Das oben unter (b) beschriebene Herstellungsverfahren für die Verbindungen der Formel (I) wird vorzugsweise in Gegenwart von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Als Säureakzeptoren können bei dem erfindungsgemäßen Verfahren (b) alle üblicherweise für derartige Umsetzungen verwendbaren Saurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man je Mol Ausgangsstoff der Formel (V) im allgemeinen 1,0 bis 1,5 Mol, vorzugsweise 1,0 bis 1,2 Mol Ausgangsstoff der Formel (IV) ein.

Die Ausgangsstoffe der Formeln (IV) und (V) und gegebenenfalls der Säureakzeptor und das Verdünnungsmittel werden im allgemeinen bei Raumtemperatur oder unter leichter Aussenkühlung zusammengegeben und das Reaktionsgemisch wird gegebenenfalls bei erhöhter Temperatur bis zum Reaktionsende gerührt. Die Aufarbeitung und Isolierung der neuen Verbindungen der Formel (I) kann nach üblichen Methoden durchgeführt werden.

Das oben unter (c) beschriebene Herstellungsverfahren für die Verbindungen der Formel (I) wird vorzugsweise in Gegenwart von Verdünnungsmitteln durchgeführt. Es kommen hierbei die gleichen organischen Lösungsmittel in Betracht, die oben im Zusammnhang mit der Beschreibung des erfindungsgemäßen Verfahrens (a) genannt wurden.

Das erfindungsgemäße Verfahren (c) wird vorzugsweise in Gegenwart eines Säureakzeptors durchgeführt. Als solche kommen hierbei die gleichen anorganischen Säurebindemittel und organischen Basen in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen -20 °C und +100 °C, vorzugsweise bei Temperaturen zwischen 0 °C und 50 °C. Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man je Mol Ausgangsstoff der Formel (VI) im allgemeinen 1,0 bis 1,5 Mol, vorzugsweise 1,0 bis 1,2 Mol Ausgangsstoff der Formel (III) ein.

Die Durchführung und Aufarbeitung kann bei Verfahren (c), wie oben für Verfahren (b) beschrieben, erfolgen.

Bei der Durchführung des erfindungsgemäßen Verfahrens (d) können als Verdünnungsmittel vorzugsweise polare organische Lösungsmittel eingesetzt werden. Bevorzugt in Betracht kommen hierbei Ketone, wie z. B. Aceton, Methylethylketon, Methylisopropylketon und Methylisobutylketon, ferner Nitrile, außerdem Amide, wie Dimethylformamid, Dimethylacetamid und N-Methyl-pyrrolidon, sowie Ether, wie Diethyl- und Dibutyl-ether, Glykoldimethylether und Diglykoldimethylether, Tetrahydrofuran und Dioxan.

Als Säureakzeptoren können bei der Durchführung des erfindungsgemäßen Verfahrens (d) vorzugsweise stark basische, jedoch schwach nucleophile Stoffe eingesetzt werden. Vorzugsweise in Frage kommen Alkalimetallhydride wie z. B. Natrium- und Kaliumhydrid, Erdalkalihydride wie z. B. Calciumhydrid, Alkalicarbonate und -alkoholate wie Natrium-und Kaliumcarbonat, Natrium- und Kaliummethylat bzw. -ethylat und Kalium-tert.-butylat, ferner 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperatur kann beim erfindungsgemäßen Verfahren (d) zur Herstellung der neuen Verbindungen der Formel (I) innerhalb eines größeren Bereiches variiert werden. Im allgemeinen arbeitet man bei Temperaturen zwischen 0 °C und +100 °C, vorzugsweise zwischen 0 °C und 80 °C. Das erfindungsgemäße Verfahren (d) wird im allgemeinen unter Normaldruck durchgeführt.

Zur Durchführung des erfindungsgemäßen Verfahrens (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden.

Die erfindungsgemäßen Verbindungen der Forml (I) können nach üblichen Methoden in Salze mit Metallen überführt werden. Hierzu werden die Verbindungen der Formel (I) im allgemeinen in inerten Lösungsmitteln, wie sie oben für die Herstellungsverfahren (a) bis (d) angegeben sind, dispergiert und mit angenähert äquimolaren Mengen von löslichen Metallverbindungen, wie z. B. Natriummethylat, Kalium-ethylat oder Calciumhydroxid, umgesetzt und bei Raumtemperatur gerührt.

Soweit die Salze hierbei kristallin anfallen, werden sie durch Absaugen isoliert. Andernfalls erhält man sie nach Abdestillieren der Lösungsmittel in kristalliner Form.

Die erfindungsgemäßen Wirkstoffe können als Defoliants, Desiccants, Krautabtötungsmittel und insbesondere als Unkrautvernichtungsmittel verwendet werden Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen bei sehr hoher Wirkung gegen dikotyole Unkräuter gute Verträglichkeit in monokotylen Kulturen, wie insbesondere Weizen, Gerste, Reis und Mais, und zeigen zudem praktisch keine Persistenz, da sie sehr schnell abgebaut werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 2-Chlor-N-{[4-methoxy-6-methyl-1,3,5-triazin-2-yl)-amino]-carbonyl}-benzolsulfonamid, Ethyl-2-{[(4-Chlor-6-methoxy-2-pyrimidyl)aminocarbonyl]aminosulfonyl}-benzoat, 2-Ethylamino-6 (1,1-dimethylethyl-amino)-4-methylthio-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 5-(2-Chlor-4-trifluormethyl-phenoxy)-N-methylsulfonyl-2-nitrobenzamid, (2-Ethoxy-1-methyl-2-oxo-ethyl)-5-(2-chlor-4-trifluormethyl-phenoxy)-2-nitrobenzoat, 2-[4-(2,4-Dichlorphenoxy)-phenoxy]-propionsäuremethylester, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, 2-[5-Methyl-5-(1-methylethyl)-4-oxo-2-imidazolin-2-yl]-3-pyridincarbonsäure, 2-(1-Ethoxyamino-butyliden)-5-(2-ethylthiopropyl)-1,3-cyclohexandion, [(4-Amino-3,5-dichlor-6-fluor-2-pyridyl)oxy]-essigsäure und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

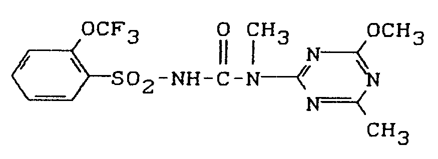

### (Verfahrensvariante (d))

4,1 g (0,01 Mol) 3-(4-Methoxy-6-methyl-1,3,5-triazin-2-yl)-1-(2-trifluormethoxy-benzolsulfonyl)-harnstoff werden in 50 ml Tetrahydrofuran aufgenommen und nacheinander mit 2,4 g (0,02 Mol) Kalium-tert.-butylat und 1,7 g (0,018 Mol) Dimethylsulfat versetzt. Nach 2-stündigem Rühren bei 25 °C wird eingeengt, mit 10 %iger Salzsäure angesäuert und mit Ethanol versetzt. Das kristallin anfallende Produkt wird abgesaugt und getrocknet.

Man erhält 3,0 g (71 % der Theorie) 3-Methyl-3-(4-methoxy-6-methyl-1,3,5-triazin-2-yl)-1-(2-trifluormethoxy-benzolsulfonyl)-harnstoff als weiße Kristalle vom Schmelzpunkt 162 °C.

Nach den oben beschriebenen Verfahrensvarianten (a) bis (d) können auch die in der nachstehenden Tabelle 1 aufgeführten Verbindungen der Formel (I) hergestellt werden:

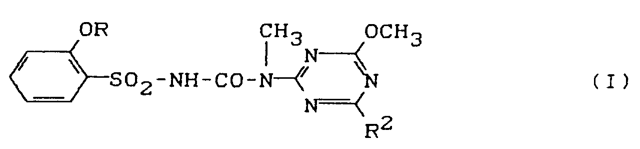

**Tabelle 1**

| Beispiel Nr. | R | R² | Schmelzpunkt (°C) |
|---|---|---|---|
| 2 | CHF₂ | CH₃ | 156 |
| 3 | CHF₂ | OCH₃ | - |
| 4 | CF₃ | OCH₃ | 138 |

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung
Die erfindungsgemäßen Wirkstoffe gemäß den Herstellungsbeispielen 1, 2, 3 und 4 zeigen in diesem Test eine sehr gute herbizide Wirksamkeit, beispielsweise bei der selektiven Bekämpfung von Abutilon, Datura, Sinapis und Stellaria in Weizen.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
O % = keine Wirkung (wie unbehandelte Kontrolle)
100 % = totale Vernichtung
Die erfindungsgemäßen Wirkstoffe gemäß Herstellungsbeispielen 1 und 4 zeigen in diesem Test eine sehr gute herbizide Wirksamkeit, beispielsweise bei der selektiven Bekämpfung von Datura, Galinsoga, Ipomoea, Matricaria, Stellaria und Lolium in Weizen.

## Patentansprüche (Patentansprüche für folgende(n) Vertragsstaat(en): AT, BE, CH, DE, FR, GB, IT, LI, NL, SE)

1. 3-Methyl-3-(1,3,5-triazin-2-yl)-1-(2-fluormethoxy-benzolsulfonyl)-harnstoffe der Formel (I), 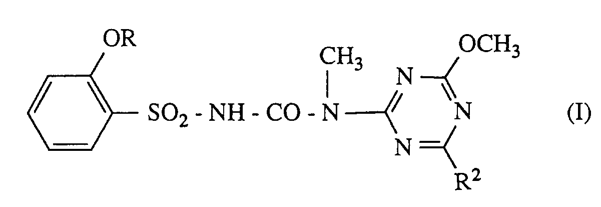 in welcher
R für CHF₂ oder CF₃ steht und
R² für CH₃ oder OCH₃ steht,
sowie Salze von Verbindungen der Formel (I) mit Natrium, Kalium, Magnesium oder Calcium.

2. Verfahren zur Herstellung von 3-Methyl-3-(1,3,5-triazin-2-yl)-1-(2-fluormethoxy-benzolsulfonyl)-harnstoffen der Formel (I) gemäß Anspruch 1, dadurch gekennzeichnet, daß man
(a) 2-Fluormethoxy-benzolsulfonyl-isocyanate der Formel (II), 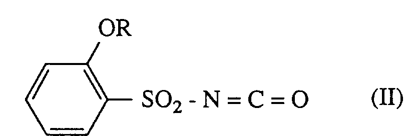 in welcher
R für CHF₂ oder CF₃ steht,
mit N-Methyl-(1,3,5-triazin-2-yl)-aminen der Formel (III), 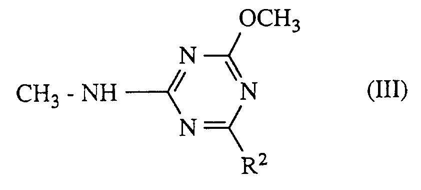 in welcher
R² für CH₃ oder OCH₃ steht,
gegebenenfalls in Gegenwart von Verdünnungsmitteln und gegebenenfalls in Gegenwart von Katalysatoren umsetzt und gegebenenfalls die so erhaltenen Produkte nach üblichen Methoden in die Na-, K-, Mg- oder Ca-Salze überführt,
oder daß man
(b) 2-Fluormethoxy-benzolsulfonsäureamide der Formel (IV), 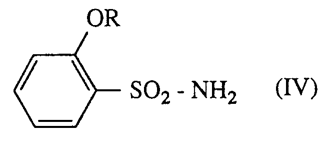 in welcher
R für CHF₂ oder CF₃ steht,
mit N-Methyl-N-(1,3,5-triazin-2-yl)-urethanen der Formel (V) 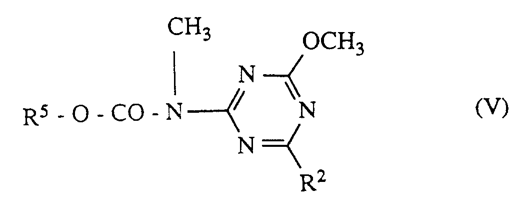 in welcher
R² für CH₃ oder OCH₃ steht und
R⁵ für C₁-C₄-Alkyl, Benzyl oder Phenyl steht,
gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt,
oder daß man
(c) N-(2-Fluormethoxy-benzolsulfonyl)-urethane der Formel (VI), 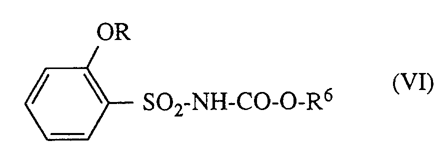 in welcher
R für CHF₂ oder CF₃ steht und
R⁶ für C₁-C₄-Alkyl, Benzyl oder Phenyl steht,
mit N-Methyl-(1,3,5-triazin-2-yl)-aminen der Formel (III), 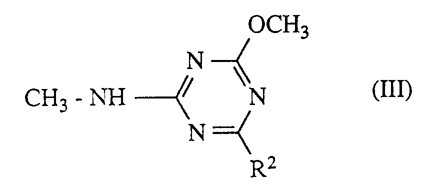 in welcher
R² für CH₃ oder OCH₃ steht,
gegebenenfalls in Gegenwart von Verdünnungsmitteln und gegebenenfalls in Gegenwart von Säureakzeptoren umsetzt und gegebenenfalls, die so erhaltenen Produkte nach üblichen Methoden in die Na-, K-, Mg- oder Ca-Salze überführt, oder daß man
(d) 1-(2-Fluormethoxy-benzolsulfonyl)-3-(1,3,5-triazin-2-yl)-harnstoffe der Formel (VII), 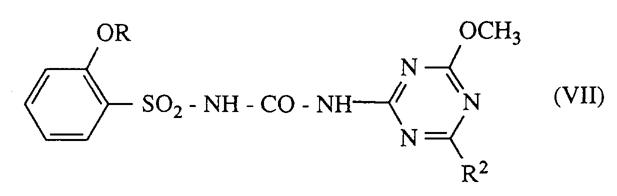 in welcher
R für CHF₂ oder CF₃ steht und
R² für CH₃ oder OCH₃ steht,
mit Verbindungen (Methylierungsmitteln) der Formel (VIII),
CH₃ - W (VIII),
in welcher
W für eine nucleophile Abgangsgruppe steht,
gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt.

3. Herbizide Mittel, gekennzeichnet durch einen Gehalt an mindestens einem 3-Methyl-3-(1,3,5-triazin-2-yl)-1-(2-fluormethoxy-benzolsulfonyl)-harnstoff der Formel (I) gemäß Anspruch 1, gegebenenfalls in Form des Na-, K-, Mg- oder Ca-Salzes.

4. Verwendung von 3-Methyl-3-(1,3,5-triazin-2-yl)-1-(2-fluormethoxy-benzolsulfonyl)-harnstoffen der Formel (I) oder von deren Na-, K-, Mg- oder Ca-Salzen gemäß Anspruch 1 zur Bekämpfüng von unerwünschtem Pflanzenwachstum.

5. Verfahren zur Herstellung von herbiziden Mitteln, dadurch gekennzeichnet, daß man 3-Methyl-3-(1,3,5-triazin-2-yl)-1-(2-fluormethoxy-benzolsulfonyl)-harnstoffe der Formel (I) oder deren Na-, K-, Mg- oder Ca-Salze gemäß Anspruch 1 mit Streckmitteln und/oder oberflächenaktiven Mitteln vermischt.

## Patentansprüche (Patentansprüche für folgende(n) Vertragsstaat(en): ES)

1. Verfahren zur Herstellung von 3-Methyl-3-(1,3,5-triazin-2-yl)-1-(2-fluormethyl-benzolsulfonyl)-harnstoffen der Formel (I), 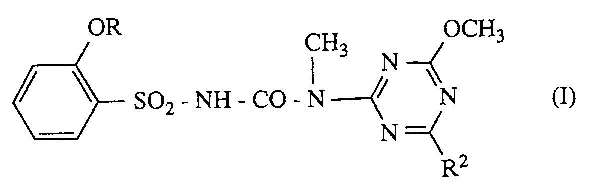 in welcher
R für CHF₂ oder CF₃ steht und
R² für CH₃ oder OCH₃ steht,
und von Salzen dieser Verbindungen mit Natrium, Kalium, Magnesium oder Calcium,
dadurch gekennzeichent, daß man
(a) 2-Fluormethoxy-benzolsulfonyl-isocyanate der Formel (II), 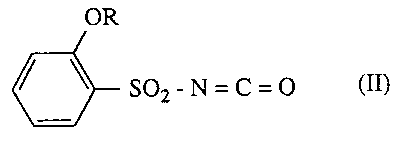 in welcher
R für CHF₂ oder CF₃ steht,
mit N-Methyl-(1,3,5-triazin-2-yl)-aminen der Formel (III), 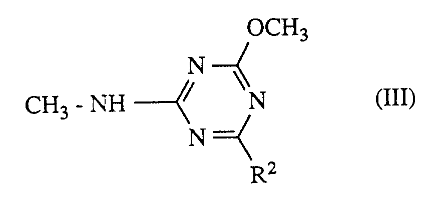 in welcher
R² für CH₃ oder OCH₃ steht,
gegebenenfalls in Gegenwart von Verdünnungsmitteln und gegebenenfalls in Gegenwart von Katalysatoren umsetzt und gegebenenfalls die so erhaltenen Produkte nach üblichen Methoden in die Na-, K-, Mg- oder Ca-Salze überführt,
oder daß man
(b) 2-Fluormethoxy-benzolsulfonsäureamide der Formel (IV), 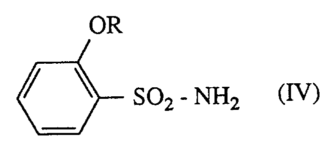 in welcher
R für CHF₂ oder CF₃ steht,
mit N-Methyl-N-(1,3,5-triazin-2-yl)-urethanen der Formel (V) 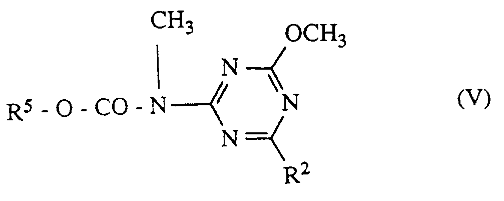 in welcher
R² für CH₃ oder OCH₃ steht und
R⁵ für C₁-C₄-Alkyl, Benzyl oder Phenyl steht,
gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt,
oder daß man
(c) N-(2-Fluormethoxy-benzolsulfonyl)-urethane der Formel (VI), 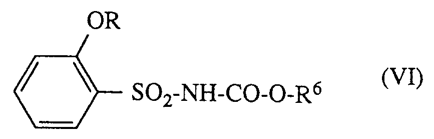 in welcher
R für CHF₂ oder CF₃ steht und
R⁶ für C₁-C₄-Alkyl, Benzyl oder Phenyl steht,
mit N-Methyl-(1,3,5-triazin-2-yl)-aminen der Formel (III), 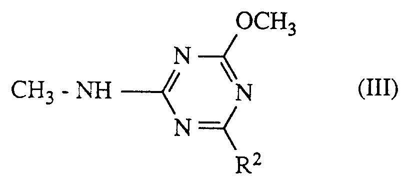 in welcher
R² für CH₃ oder OCH₃ steht,
gegebenenfalls in Gegenwart von Verdünnungsmitteln und gegebenenfalls in Gegenwart von Säureakzeptoren umsetzt und gegebenenfalls, die so erhaltenen Produkte nach üblichen Methoden in die Na-, K-, Mg- oder Ca-Salze überführt, oder daß man
(d) 1-(2-Fluormethoxy-benzolsulfonyl)-3-(1,3,5-triazin-2-yl)-harnstoffe der Formel (VII), 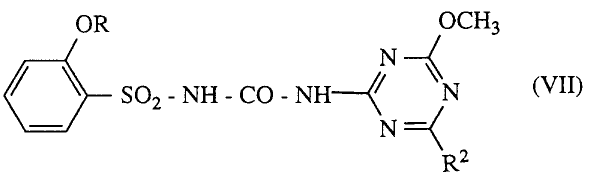 in welcher
R für CHF₂ oder CF₃ steht und
R² für CH₃ oder OCH₃ steht,
mit Verbindungen (Methylierungsmitteln) der Formel (VIII),
CH₃ - W (VIII),
in welcher
W für eine nucleophile Abgangsgruppe steht,
gegebenenfalls in Gegenwart von Verdünnungsmitteln und gegebenenfalls in Gegenwart von Säureakzeptoren umsetzt und gegebenenfalls die so erhaltenen Produkte nach üblichen Methoden in die Na-, K-, Mg- oder Ca-Salze überführt.

2. Herbizide Mittel, gekennzeichnet durch einen Gehalt an mindestens einem 3-Methyl-3-(1,3,5-triazin-2-yl)-1-(2-fluormethoxy-benzolsulfonyl)-harnstoff der Formel (I) gemäß Anspruch 1, gegebenenfalls in Form des Na-, K-, Mg- oder Ca-Salzes.

3. Verwendung von 3-Methyl-3-(1,3,5-triazin-2-yl)-1-(2-fluormethoxy-benzolsulfonyl)-harnstoffen der Formel (I) oder von deren Na-, K-, Mg- oder Ca-Salzen gemäß Anspruch 1 zur Bekämpfung von unerwünschtem Pflanzenwachstum.

4. Verfahren zur Herstellung von herbiziden Mitteln, dadurch gekennzeichnet, daß man 3-Methyl-3-(1,3,5-triazin-2-yl)-1-(2-fluormethoxy-benzolsulfonyl)-harnstoffe der Formel (I) oder deren Na-, K-, Mg- oder Ca-Salze gemäß Anspruch 1 mit Streckmitteln und/oder oberflächenaktiven Mitteln vermischt.

## Claims (Claims for the following Contracting State(s): AT, BE, CH, DE, FR, GB, IT, LI, NL, SE)

1. 3-Methyl-3-(1,3,5-triazin-2-yl)-1-(2-fluoromethoxy-benzenesulphonyl)-ureas of the formula (I) 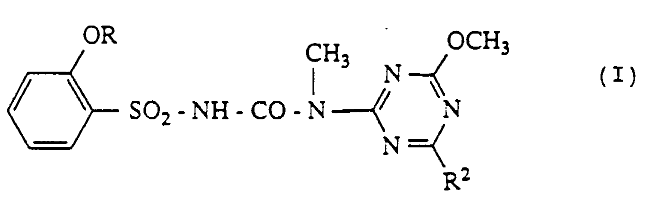 in which
R represents CHF₂ or CF₃ and
R² represents CH₃ or OCH₃,
and salts of compounds of the formula (I) with sodium, potassium, magnesium or calcium.

2. Process for the preparation of 3-methyl-3-(1,3,5-triazin-2-yl)-1-(2-fluoromethoxy-benzenesulphonyl)-ureas of the formula (I) according to Claim 1, characterised in that
(a) 2-fluoromethoxy-benzenesulphonyl isocyanates of the formula (II) 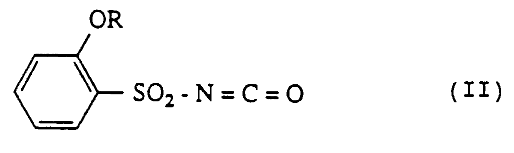 in which
R represents CHF₂ or CF₃,
are reacted with N-methyl-(1,3,5-triazin-2-yl)-amines of the formula (III) 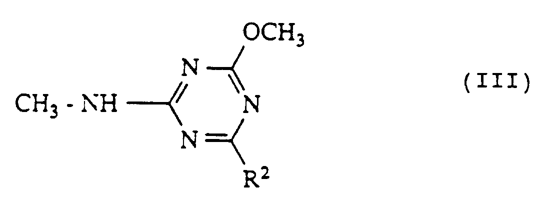 in which
R² represents CH₃ or OCH₃,
if appropriate in the presence of diluents and if appropriate in the presence of catalysts, and, if appropriate, the products thus obtained are converted into the Na, K, Mg or Ca salts by customary methods,
or in that
(b) 2-fluoromethoxy-benzenesulphonic acid amides of the formula (IV) 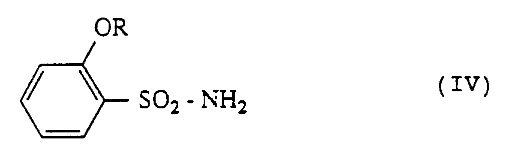 in which
R represents CHF₂ or CF₃,
are reacted with N-methyl-N-(1,3,5-triazin-2-yl)-urethanes of the formula (V) 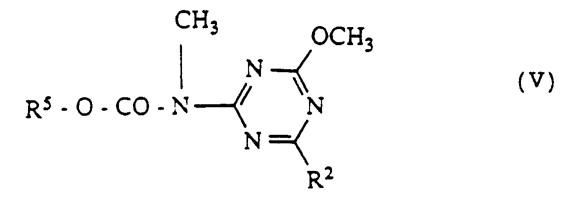 in which
R² represents CH₃ or OCH₃ and
R⁵ represents C₁-C₄-alkyl, benzyl or phenyl,
if appropriate in the presence of diluents and if appropriate in the presence of acid acceptors, and, if appropriate, the products thus obtained are converted into the Na, K, Mg or Ca salts by customary methods,
or in that
(c) N-(2-fluoromethoxy-benzenesulphonyl)-urethanes of the formula (VI) 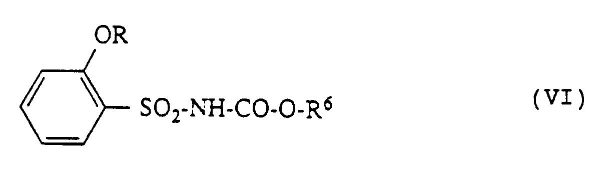 in which
R represents CHF₂ or CF₃ and
R⁶ represents C₁-C₄-alkyl, benzyl or phenyl, are reacted with N-methyl-(1,3,5-triazin-2-yl)-amines of the formula (III) 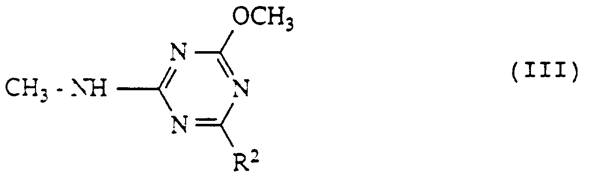 in which
R² represents CH₃ or OCH₃,
if appropriate in the presence of diluents and if appropriate in the presence of acid acceptors, and, if appropriate, the products thus obtained are converted into the Na, K, Mg or Ca salts by customary methods, or in that
(d) 1-(2-fluoromethoxy-benzenesulphonyl)-3-(1,3,5-triazin-2-yl)-ureas of the formula (VII) 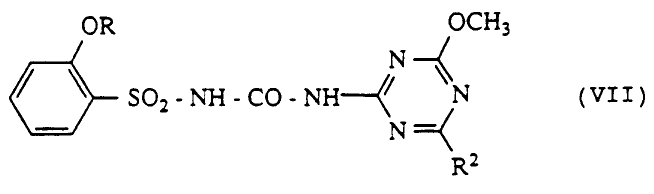 in which
R represents CHF₂ or CF₃ and
R² represents CH₃ or OCH₃,
are reacted with compounds (methylating agents) of the formula (VIII),
CH₃-W (VIII),
in which
W represents a nucleophilic leaving group,
if appropriate in the presence of diluents and if appropriate in the presence of acid acceptors, and, if appropriate, the products thus obtained are converted into the Na, K, Mg or Ca salts by customary methods.

3. Herbicidal agents, characterised in that they contain at least one 3-methyl-3-(1,3,5-triazin-2-yl)-1-(2-fluoromethoxy-benzenesulphonyl)-urea of the formula (I) according to Claim 1, if appropriate in the form of the Na, K, Mg or Ca salt.

4. Use of 3-methyl-3-(1,3,5-triazin-2-yl)-1-(2-fluoromethoxy-benzenesulphonyl)-ureas of the formula (I) or of Na, K, Mg or Ca salts thereof according to Claim 1 for combating undesirable plant growth.

5. Process for the preparation of herbicidal agents, characterised in that 3-methyl-3-(1,3,5-triazin-2-yl)-1-(2-fluoromethoxy-benzenesulphonyl)-ureas of the formula (I) or Na, K, Mg or Ca salts thereof according to Claim 1 are mixed with extenders and/or surface-active agents.

## Claims (Claims for the following Contracting State(s): ES)

1. Process for the preparation of 3-methyl-3-(1,3,5-triazin-2-yl)-1-(2-fluoromethyl-benzenesulphonyl)-ureas of the formula (I) 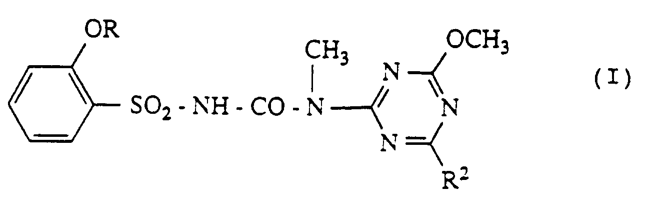 in which
R represents CHF₂ or CF₃ and
R² represents CH₃ or OCH₃,
and of salts of these compounds with sodium, potassium, magnesium or calcium,
characterised in that
(a) 2-fluoromethoxy-benzenesulphonyl isocyanates of the formula (II) 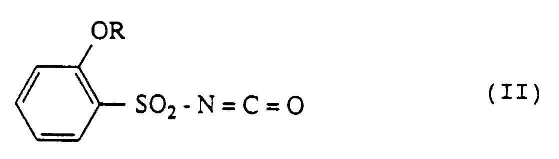 in which
R represents CHF₂ or CF₃,
are reacted with N-methyl-(1,3,5-triazin-2-yl)-amines of the formula (III) 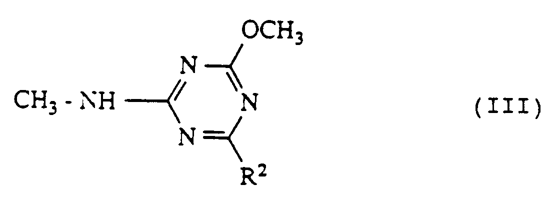 in which
R² represents CH₃ or OCH₃,
if appropriate in the presence of diluents and if appropriate in the presence of catalysts, and, if appropriate, the products thus obtained are converted into the Na, K, Mg or Ca salts by customary methods,
or in that
(b) 2-fluoromethoxy-benzenesulphonic acid amides of the formula (IV) 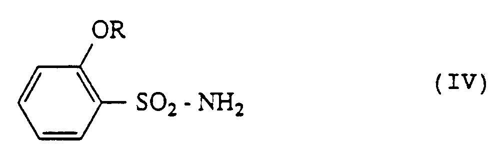 in which
R represents CHF₂ or CF₃,
are reacted with N-methyl-N-(1,3,5-triazin-2-yl)-urethanes of the formula (V) 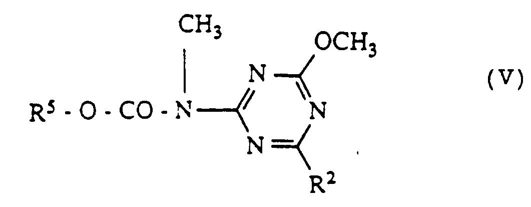 in which
R² represents CH₃ or OCH₃ and
R⁵ represents C₁-C₄-alkyl, benzyl or phenyl,
if appropriate in the presence of diluents and if appropriate in the presence of acid acceptors, and, if appropriate, the products thus obtained are converted into the Na, K, Mg or Ca salts by customary methods,
or in that
(c) N-(2-fluoromethoxy-benzenesulphonyl)-urethanes of the formula (VI) 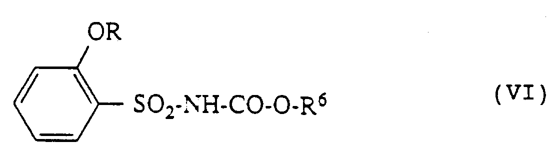 in which
R represents CHF₂ or CF₃ and
R⁶ represents C₁-C₄-alkyl, benzyl or phenyl, are reacted with N-methyl-(1,3,5-triazin-2-yl)-amines of the formula (III) 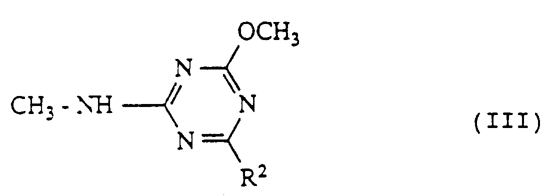 in which
R² represents CH₃ or OCH₃,
if appropriate in the presence of diluents and if appropriate in the presence of acid acceptors, and, if appropriate, the products thus obtained are converted into the Na, K, Mg or Ca salts by customary methods, or in that
(d) 1-(2-fluoromethoxy-benzenesulphonyl)-3-(1,3,5-triazin-2-yl)-ureas of the formula (VII) 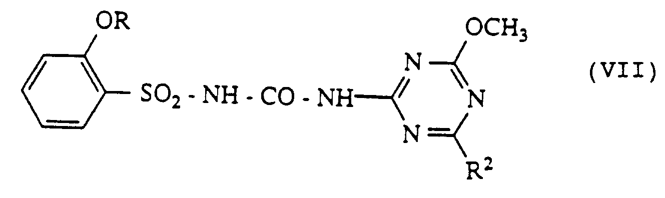 in which
R represents CHF₂ or CF₃ and
R² represents CH₃ or OCH₃,
are reacted with compounds (methylating agents) of the formula (VIII)
CH₃-W (VIII)
in which
W represents a nucleophilic leaving group,
if appropriate in the presence of diluents and if appropriate in the presence of acid acceptors, and, if appropriate, the products thus obtained are converted into the Na, K, Mg or Ca salts by customary methods.

2. Herbicidal agents, characterised in that they contain at least one 3-methyl-3-(1,3,5-triazin-2-yl)-1-(2-fluoromethoxy-benzenesulphonyl)-urea of the formula (I) according to Claim 1, if appropriate in the form of the Na, K, Mg or Ca salt.

3. Use of 3-methyl-3-(1,3,5-triazin-2-yl)-1-(2-fluoromethoxy-benzenesulphonyl)-ureas of the formula (I) or of Na, K, Mg or Ca salts thereof according to Claim 1 for combating undesirable plant growth.

4. Process for the preparation of herbicidal agents, characterised in that 3-methyl-3-(1,3,5-triazin-2-yl)-1-(2-fluoromethoxy-benzenesulphonyl)-ureas of the formula (I) or Na, K, Mg or Ca salts thereof according to Claim 1 are mixed with extenders and/or surface-active agents.

## Revendications (Revendications pour l'(les) Etat(s) contractant(s) suivant(s): AT, BE, CH, DE, FR, GB, IT, LI, NL, SE)

1. 3-méthyl-3-(1,3,5-triazin-2-yl)-1-(2-fluorométhoxy-benzènesulfonyl)-urées de formule (I) 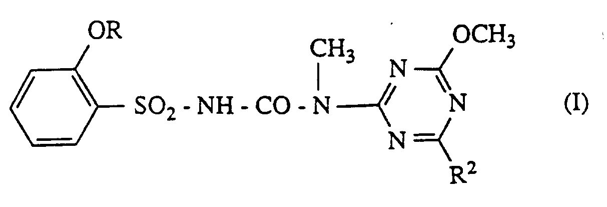 dans laquelle
R représente CHF₂ ou CF₃ et
R² représente CH₃ ou OCH₃,
ainsi que les sels des composés de formule (I) avec le sodium, le potassium, le magnésium ou le calcium.

2. Procédé pour la préparation de 3-méthyl-3-(1,3,5-triazin-2-yl)-1-(2-fluorométhoxy-benzènesulfonyl)-urées de formule (I) selon la revendication 1, caractérisé en ce qu'on fait réagir
(a) des 2-fluorométhoxybenzènesulfonylisocyanates de formule (II) 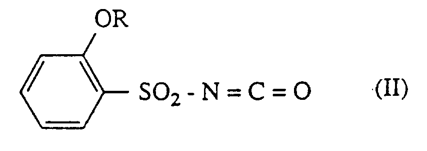 dans laquelle
R représente CHF₂ ou CF₃,
avec des N-méthyl-(1,3,5-triazin-2-yl)-amines de formule (III) 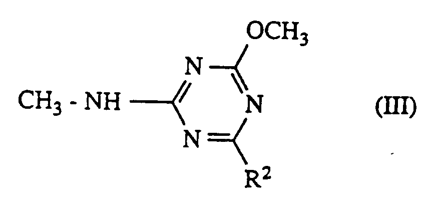 dans laquelle
R² représente CH₃ ou OCH₃,
éventuellement en présence de diluants et éventuellement en présence de catalyseurs, et on transforme éventuellement d'après des procédés habituels les produits ainsi obtenus en sels de Na, K, Mg ou Ca,
ou bien en ce qu'on fait réagir
(b) des 2-fluorométhoxybenzènesulfonamides de formule (IV) 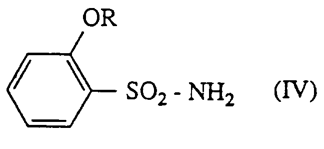 dans laquelle
R représente CHF₂ ou CF₃,
avec des N-méthyl-N-(1,3,5-triazin-2-yl)-uréthannes de formule (V) 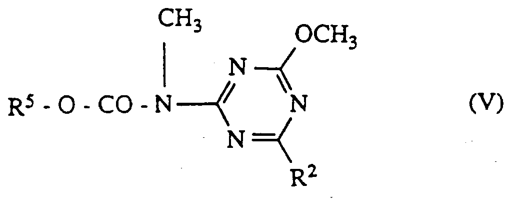 dans laquelle
R² représente CH₃ ou OCH₃ et
R⁵ représente un groupe alkyle en C₁-C₄, un groupe benzyle ou un groupe phényle,
éventuellement en présence de diluants et éventuellement en présence d'accepteurs d'acides, et on transforme éventuellement d'après des procédés habituels les produits ainsi obtenus en sels de Na, K, Mg ou Ca,
ou bien en ce qu'on fait réagir
(c) des N-(2-fluorométhoxybenzènesulfonyl)-uréthannes de formule (VI) 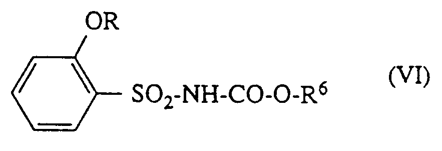 dans laquelle
R représente CHF₂ ou CF₃ et
R⁶ représente un groupe alkyle en C₁-C₄, un groupe benzyle ou un groupe phényle,
avec des N-méthyl-(1,3,5-triazin-2-yl)-amines de formule (III) 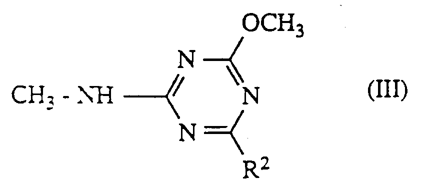 dans laquelle
R² représente CH₃ ou OCH₃,
éventuellement en présence de diluants et éventuellement en présence d'accepteurs d'acides, et on transforme éventuellement d'après des procédés habituels les produits ainsi obtenus en sels de Na, K, Mg ou Ca,
ou bien en ce qu'on fait réagir
(d) des 1-(2-fluorométhoxybenzènesulfonyl)-3-(1,3,5-triazin-2-yl)-urées de formule (VII) 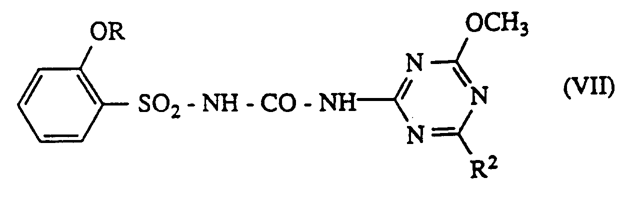 dans laquelle
R représente CHF₂ ou CF₃ et
R² représente CH₃ ou OCH₃,
avec des composés (agents de méthylation) de formule (VIII)
CH₃-W (VIII)
dans laquelle
W représente un groupe nucléophile qui se sépare,
éventuellement en présence de diluants et éventuellement en présence d'accepteurs d'acides, et on transforme éventuellement d'après des procédés habituels les produits ainsi obtenus en sels de Na, K, Mg ou Ca.

3. Agents herbicides caractérisés par une teneur en au moins une 3-méthyl-3-(1,3,5-triazin-2-yl)-1-(2-fluorométhoxy-benzènesulfonyl)-urée de formule (I) selon la revendication 1, éventuellement sous forme du sel de Na, K, Mg ou Ca.

4. Utilisation de 3-méthyl-3-(1,3,5-triazin-2-yl)-1-(2-fluorométhoxy-benzènesulfonyl)-urées de formule (I) ou de leurs sels de Na, K, Mg ou Ca selon la revendication 1, pour lutter contre la croissance de plantes non désirées.

5. Procédé pour la préparation d'agents herbicides, caractérisé en ce qu'on mélange des 3-méthyl-3-(1,3,5-triazin-2-yl)-1-(2-fluorométhoxy-benzènesulfonyl)-urées de formule (I) ou leurs sels de Na, K, Mg ou Ca selon la revendication 1 avec des diluants et/ou des agents tensioactifs.

## Revendications (Revendications pour l'(les) Etat(s) contractant(s) suivant(s): ES)

1. Procédé pour la préparation de 3-méthyl-3-(1,3,5-triazin-2-yl)-1-(2-fluorométhoxy-benzènesulfonyl)-urées de formule (I), 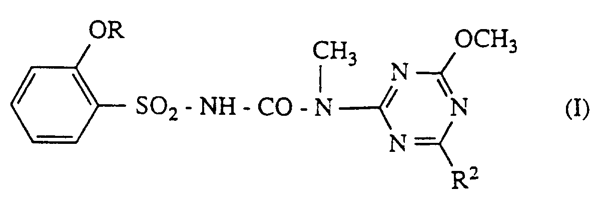 dans laquelle
R représente CHF₂ ou CF₃ et
R² représente CH₃ ou OCH₃,
et des sels de ces composés avec le sodium, le potassium, le magnésium ou le calcium,
caractérisé en ce qu'on fait réagir
(a) des 2-fluorométhoxybenzènesulfonylisocyanates de formule (II) 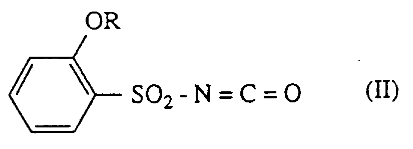 dans laquelle
R représente CHF₂ ou CF₃,
avec des N-méthyl-(1,3,5-triazin-2-yl)-amines de formule (III) 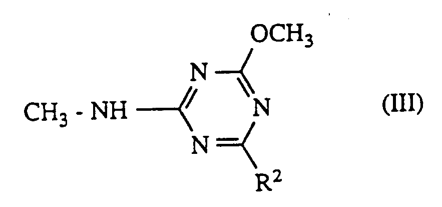 dans laquelle
R² représente CH₃ ou OCH₃,
éventuellement en présence de diluants et éventuellement en présence de catalyseurs, et on transforme éventuellement d'après des procédés habituels les produits ainsi obtenus en sels de Na, K, Mg ou Ca,
ou bien en ce qu'on fait réagir
(b) des 2-fluorométhoxybenzènesulfonamides de formule (IV) 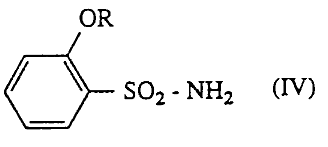 dans laquelle
R représente CHF₂ ou CF₃,
avec des N-méthyl-N-(1,3,5-triazin-2-yl)-uréthannes de formule (V) 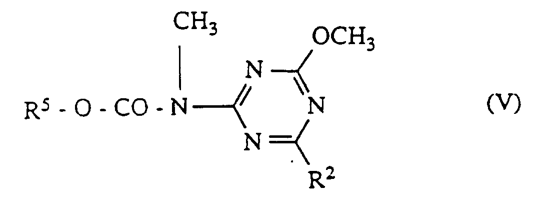 dans laquelle
R² représente CH₃ ou OCH₃ et
R⁵ représente un groupe alkyle en C₁-C₄, un groupe benzyle ou un groupe phényle,
éventuellement en présence de diluants et éventuellement en présence d'accepteurs d'acides, et on transforme éventuellement d'après des procédés habituels les produits ainsi obtenus en sels de Na, K, Mg ou Ca,
ou bien en ce qu'on fait réagir
(c) des N-(2-fluorométhoxybenzènesulfonyl)-uréthannes de formule (VI) 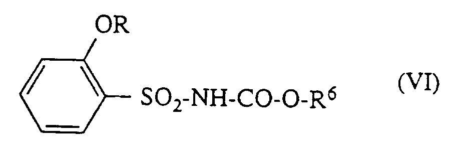 dans laquelle
R représente CHF₂ ou CF₃ et
R⁶ représente un groupe alkyle en C₁-C₄, un groupe benzyle ou un groupe phényle,
avec des N-méthyl-(1,3,5-triazin-2-yl)-amines de formule (III) 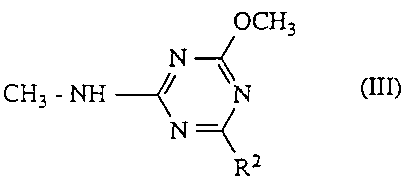 dans laquelle
R² représente CH₃ ou OCH₃,
éventuellement en présence de diluants et éventuellement en présence d'accepteurs d'acides, et on transforme éventuellement d'après des procédés habituels les produits ainsi obtenus en sels de Na, K, Mg ou Ca,
ou bien en ce qu'on fait réagir
(d) des 1-(2-fluorométhoxybenzènesulfonyl)-3-(1,3,5-triazin-2-yl)-urées de formule (VII) 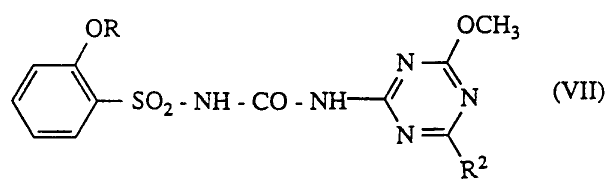 dans laquelle
R représente CHF₂ ou CF₃ et
R² représente CH₃ ou OCH₃,
avec des composés (agents de méthylation) de formule (VIII)
CH₃-W (VIII)
dans laquelle
W représente un groupe nucléophile qui se sépare,
éventuellement en présence de diluants et éventuellement en présence d'accepteurs d'acides, et on transforme éventuellement d'après des procédés habituels les produits ainsi obtenus en sels de Na, K, Mg ou Ca.

2. Agents herbicides caractérisés par une teneur en au moins une 3-méthyl-3-(1,3,5-triazin-2-yl)-1-(2-fluorométhoxy-benzènesulfonyl)-urée de formule (I) selon la revendication 1, éventuellement sous forme du sel de Na, K, Mg ou Ca.

3. Utilisation de 3-méthyl-3-(1,3,5-triazin-2-yl)-1-(2-fluorométhoxy-benzènesulfonyl)-urées de formule (I) ou de leurs sels de Na, K, Mg ou Ca selon la revendication 1, pour lutter contre la croissance de plantes non désirées.

4. Procédé pour la préparation d'agents herbicides, caractérisé en ce qu'on mélange des 3-méthyl-3-(1,3,5-triazin-2-yl)-1-(2-fluorométhoxy-benzènesulfonyl)-urées de formule (I) ou leurs sels de Na, K, Mg ou Ca selon la revendication 1 avec des diluants et/ou des agents tensioactifs.
